# EUROPÄISCHE PATENTSCHRIFT

(11) **EP 2 304 432 B1**
(45) Veröffentlichungstag und Bekanntmachung des Hinweises auf die Patenterteilung: **14.03.2012**
(21) Anmeldenummer: 09769192.7
(22) Anmeldetag: 19.06.2009
(51) Int. Cl.: G01N 33/487

(54) **ANALYSEHANDGERÄT ZUR UNTERSUCHUNG EINER KÖRPERFLÜSSIGKEIT UND STEUERVERFAHREN DAFÜR**
HAND-HELD ANALYSIS APPLIANCE FOR ANALYSING A BODY FLUID, AND METHOD FOR CONTROLLING SAID APPLIANCE
APPAREIL À MAIN D'ANALYSE POUR L'EXAMEN D'UN LIQUIDE CORPOREL ET PROCÉDÉ DE COMMANDE DE CET APPAREIL

(30) Priorität: 25.06.2008 EP 08158943
(43) Veröffentlichungstag der Anmeldung: 06.04.2011
(73) Patentinhaber: F. Hoffmann-La Roche AG, 4070 Basel (CH); Roche Diagnostics GmbH, 68305 Mannheim (DE)
(72) Erfinder: MILTNER, Karl, 67227 Frankenthal (DE); LIEDTKE, Sebastian, 69117 Heidelberg (DE); BAETER, Thorsten, 67136 Fußgönheim (DE); SCHMID, Wilfried, Dr., 68165 Mannheim (DE); FRISCH, Gerhard, 68535 Edingen-Neckarhausen (DE); HECK, Wolfgang, 67227 Frankenthal (DE)
(74) Vertreter: Pfiz, Thomas
(86) Internationale Anmeldenummer: PCT/EP2009/057666
(87) Internationale Veröffentlichungsnummer: WO 2009/156343

(56) Entgegenhaltungen:
- EP-A- 1 770 395
- EP-A- 1 889 570
- WO-A-2007/090662
- WO-A-2008/022999
- US-A- 4 685 059

## Beschreibung

Die Erfindung betrifft ein Verfahren zur Steuerung eines Analysehandgeräts zur Untersuchung einer Körperflüssigkeit, insbesondere für Blutzuckertests, bei dem mittels einer geräteseitigen Steuereinrichtung in aufeinanderfolgenden Messzyklen jeweils mindestens ein Testmittel für den Einmalgebrauch automatisch bereitgestellt wird, wobei jeder Messzyklus in einem Messbetrieb der Steuereinrichtung durch eine Startaktuation ausgelöst wird. Die Erfindung betrifft weiter ein Analysehandgerät zur Untersuchung einer Körperflüssigkeit.

Ein solches Verfahren geht aus der EP-A 1 770 395 hervor, bei dem einzelne Teststreifen aus einem wechselbaren Trommelmagazin für Analysezwecke bereitgestellt werden. Dort erfolgt durch Zuordnen eines Zählerstandes zu einer Magazinkennung eine geräteseitige Verbrauchserfassung unter Rückschreibeverzicht auf das Magazin, so dass auch der wahlweise Einsatz von teilverbrauchten Magazinen vereinfacht wird.

Zur weiteren Systemintegration wurde beispielsweise in der EP-A 1 739 432 bereits vorgeschlagen, anstelle einzelner Streifen ein aufgewickeltes Testband mit beabstandeten analytischen Testfeldern als Testmittel in einem Handgerät einzusetzen. Zur Wegerfassung beim Bandtransport sind dort Markierungsbereiche vorgesehen, die eine exakte Positionierung der Testfelder erlauben. Bei solchen Konzepten stellt die Minimierung der Baugröße und der erforderlichen Bedienschritte eine besondere Herausforderung dar.

Aus der WO 2008/022999 ist ein Analysehandgerät mit Testband-kassette bakannt, wobei eine kassettenseitige Sperrvezahnung in der Eingriffstellung den Bandtransport vehrindert, um so ungewollten Testmittelverlust zu vermeisten.

Ausgehend hiervon liegt der Erfindung die Aufgabe zugrunde, die im Stand der Technik bekannten Verfahren und Erzeugnisse weiter zu entwickeln und mit einfachen Mitteln eine erhöhte Benutzerfreundlichkeit und Verfahrenssicherheit auch hinsichtlich einer optimierten Nutzung der Testmittel zu erreichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von der Überlegung aus, dass die Bereitstellung eines analytischen Testmittels in einem Handgerät in der Regel nicht rückgängig gemacht werden kann, ohne die Testqualität zu mindern. Diese Problematik ist vergleichbar mit herkömmlichen Filmkameras, die bei eingelegtem Film nicht einfach geöffnet werden sollten. Dementsprechend wird in verfahrensmäßiger Hinsicht vorgeschlagen, dass die geräteseitig implementierte, programmgesteuerte Steuereinrichtung durch einen definierten Steuereingriff in einen Wartungsbetrieb versetzt wird, in welchem die automatische Bereitstellung eines in das Gerät eingewechselten disposiblen Testmittels verhindert wird. Auf diese Weise wird ein ungewollter Testverlust vermieden, ohne dass die Handhabung im normalen Messablauf beeinträchtigt würde.

Vorteilhafterweise wird der Start eines Messzyklus im Wartungsbetrieb auch bei einer Startaktuation blockiert. Somit können Handhabungsschritte vorgenommen werden, die im Messbetrieb zu einem Start der Messung führen würden.

Zweckmäßig ist der Wartungsbetrieb dazu eingerichtet, eine Gerätereinigung oder einen Testmittelaustausch bzw. ein zeitweises Herausnehmen des Testmittels aus dem Gerät ohne Testmittelverlust durch eine ungewollte Testbereitstellung zu ermöglichen.

Um den Bauaufwand zu reduzieren, ist es vorteilhaft, wenn der Wartungsbetrieb über ein Softwaremenü der elektronischen Steuereinrichtung durch einen Benutzer ausgewählt wird. Dies lässt sich besonders einfach dadurch realisieren, dass der Wartungsbetrieb über einen manuell betätigbaren Schalter, insbesondere einen Softkey eingeleitet wird.

Eine weitere vorteilhafte Ausgestaltung sieht vor, dass eine vorbestimmte Betätigung eines Gerätebauteils durch die Steuereinrichtung als Startaktuation erkannt wird. Auf diese Weise kann die Anzahl der erforderlichen Bedienschritte reduziert werden und eine schnelle Verfügbarkeit erreicht werden. In diesem Zusammenhang ist es auch günstig, wenn die Startaktuation und/oder ein Austausch der Testmittel durch einen Sensor überwacht wird, und wenn im Wartungsbetrieb das Sensorsignal ignoriert wird.

Vorteilhafterweise werden die Testmittel in einem Gehäuse des Analysehandgeräts gegen Umwelteinflüsse, insbesondere Feuchtigkeitszutritt geschützt, um eine hohe Zuverlässigkeit zu gewährleisten.

Eine weitere Verbesserung der Bedienfreundlichkeit lässt sich dadurch erreichen, dass die Messzyklen durch das Öffnen einer Schutzabdeckung des Analysehandgeräts ausgelöst werden, wobei ein Testmittel für einen Benutzerzugriff bereitgestellt wird.

Vor allem für den Einsatz von magazinierten Testmitteln ist es vorteilhaft, wenn die Testmittel im Messbetrieb durch eine Transporteinrichtung an mindestens eine vorgegebene Position transportiert werden, und wenn der automatische Testmitteltransport im Wartungsbetrieb unterbrochen wird. Gemäß einer besonderen Ausgestaltung werden während eines Messzyklus die jeweiligen Testmittel von einem Testmittelvorrat zu einer Applikationsstelle und gegebenenfalls zu einem Testmittelabfall transportiert.

Eine besonders vorteilhafte Anwendung besteht darin, dass ein mit einer Vielzahl von analytischen Testfeldern und/oder Stechelementen als Testmittel bestücktes Testband eingesetzt wird, und dass das Testband zur Bereitstellung der Testmittel abschnittsweise vorgespult wird. Eine weitere Verbesserung wird dadurch erreicht, dass die Testmittel vorzugsweise in Form eines Magazins oder einer Kassette in ein Gerätefach eingewechselt werden, und dass im Messbetrieb beim Öffnen des Gerätefachs ein durch eine Startaktuation aktiviertes Testmittel verworfen wird.

Gegenstand der Erfindung ist auch eine besondere Vorrichtung zur Durchführung des Verfahrens umfassend eine Steuereinrichtung zur automatischen Bereitstellung von für den Einmalgebrauch ausgebildeten Testmitteln in aufeinanderfolgenden Messzyklen, wobei jeder Messzyklus in einem Messbetrieb der Steuereinrichtung durch eine Startaktuation auslösbar ist, und wobei die Steuereinrichtung durch einen definierten Steuereingriff in einen Wartungsbetrieb versetzbar ist, in welchem die automatische Bereitstellung eines Testmittels verhindert wird.

Im Folgenden wird die Erfindung anhand des in der Zeichnung schematisch dargestellten Ausführungsbeispiels näher erläutert. Es zeigen:
- Fig. 1: ein tragbares Blutzuckermessgerät zum Einsatz einer Testbandkassette in perspektivischer Ansicht;
- Fig. 2: das Messgerät nach Fig. 1 in einem Längsschnitt;
- Fig. 3: ein Flussdiagramm zur Veranschaulichung des Mess- und Wartungsbetriebs des Blutzuckermessgeräts.

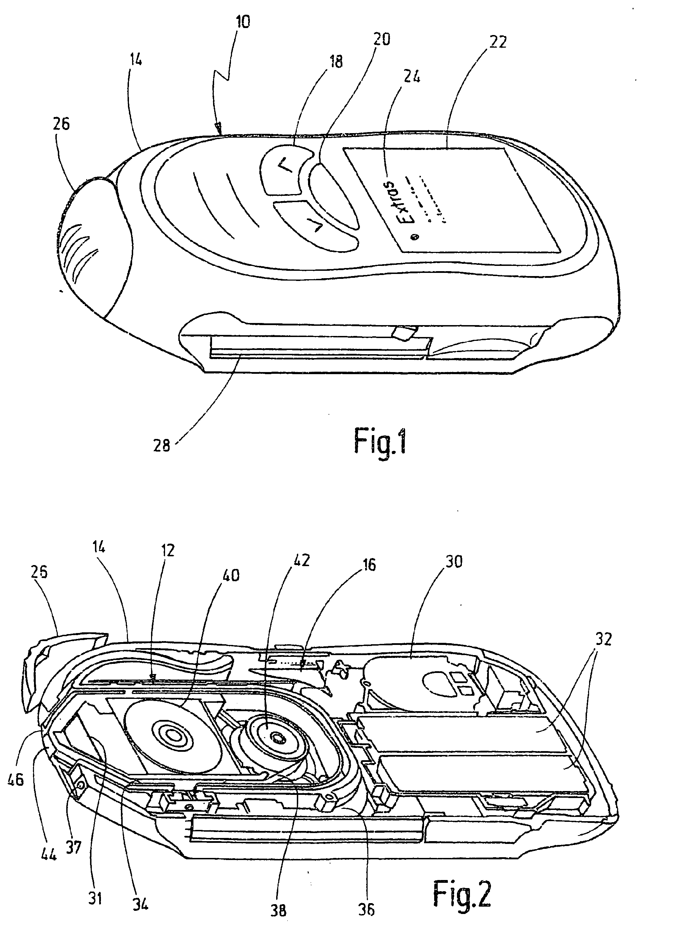

Das in Fig. 1 und 2 dargestellte tragbare Blutzuckermessgerät 10 erlaubt einem Benutzer die Selbstbestimmung des Blutzuckerspiegels vor Ort in einem weitgehend automatischen Messablauf. Dabei sind eine Vielzahl von analytischen Testmitteln in Form einer Bandkassette 12 als auswechselbare Verbrauchsmittel in das kompakte Gerätegehäuse 14 einsetzbar, um ohne aufwändige Handhabung entsprechend viele Tests durchführen zu können. Für die Gerätewartung kann ein ungewollter Testmittelverlust durch eine vorteilhafte Ablaufsteuerung mittels einer mit einer Steuerungssoftware versehenen elektronischen Steuereinrichtung 16 des Geräts 10 verhindert werden.

Wie aus Fig. 1 ersichtlich, sind an der Oberseite des Gehäuses 14 verschiedene Steuertasten 18, 20 zur manuellen Betätigung angeordnet. Ein Bildschirm 22 ermöglicht die Anzeige von Bedienmenüs 24 und von Messergebnissen. Die in dem Gehäuse 14 geschützte Bandkassette 12 ist durch Öffnen einer Schutzabdeckung 26 an einer Applikationsstelle für den Auftrag einer Blutprobe zugänglich. Eine zur Gewinnung der Blutprobe vorgesehene Stechhilfe (nicht gezeigt) lässt sich an einer Halterung 28 seitlich an dem Gehäuse 14 anflanschen.

Fig. 2 zeigt im Schnitt das Geräteinnere mit eingesetzter Bandkassette 12, Steuereinrichtung (Platine 16), Bandantriebseinheit 30, Messeinheit 31 und Energieversorgung (Batterien 32). Die in dem Kassettenfach 34 gelagerte Kassette 12 ist als Verbrauchsartikel über einen bodenseitigen Gehäusedeckel 36 auswechselbar. Die Stellung der verschiednen Gerätezugänge wird sensorisch erfasst, wie dies für die Schutzabdeckung 26 durch den Schalter 37 beispielhaft dargestellt ist.

Die Kassette 12 umfasst ein Testband 38, eine nach außen abgedichtete Vorratsspule 40 zum Abwickeln von ungebrauchtem Testband und eine Aufwickelspule 42 zum Aufwickeln von gebrauchtem Testband. Auf dem Testband 38 sind mit einer Testchemie beschichtete Testfelder 44 als Testmittel zum Nachweis des Analyten (Glukose) im Abstand voneinander aufgebracht. Alternativ oder ergänzend könnten auch nicht gezeigte Stechelemente für einen Hauteinstich auf einem Trägerband magaziniert werden. Das Testband 38 lässt sich mittels der Antriebseinheit 30 vorspulen, so dass die Testeinheiten 44 an einer durch Wegschwenken der Schutzabdeckung 26 freigegebenen Applikationsstelle 46 für die Blutprobenauftragung sukzessive bereitstellbar sind. Dabei kann durch eine rückseitige reflektometrische Verrnessung des mit Blut beaufschlagten Testfeldes 44 anhand eines Farbumschlags ein Glukosemesswert gewonnen werden.

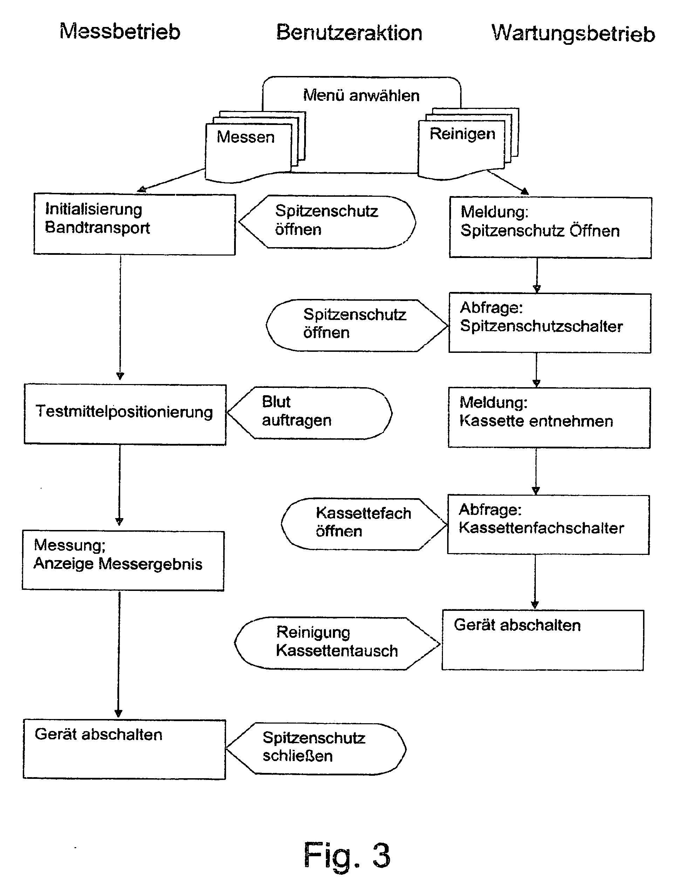

Bei der in Fig. 3 veranschaulichten Ablaufsteuerung wird durch einfache Maßnahmen ein Altemativbetrieb zwischen Messen und Wartung unter Minimierung der erforderlichen Bedienschritte bzw. Benutzeraktion ermöglicht, wobei ein Testmittelverlust weitgehend vermieden wird.

Wie im linken Teil des Diagramms nach Fig. 3 dargestellt, kann ein Messzyklus durch Öffnen des Spitzenschutzes (Schutzabdeckung 26) vom Benutzer gestartet werden, ohne dass zusätzliche Einschaltknöpfe zu bedienen wären. Damit ist eine sehr rasche Verfügbarkeit des Systems gewährleistet. Als alternative Startaktuation kann aber auch die Menüauswahl "Messen" über die Bedientasten 18, 20 gewählt werden.

Bis zum Start des Messzyklus bleiben die unbenutzten Testfelder 44 auf der nach außen abgedichteten Vorratsspule 40 gegen Umgebungseinflüsse wie Licht, Feuchte, Schmutz abgeschirmt. Die Testfeldpositionierung an der Applikationsstelle 46 wird dann durch Bandtransport gesteuert, wobei während einer Initialisierungsphase auf dem Testband 38 aufgebrachte Steuermarken erfasst werden, um ein definiertes Vorspulen zu ermöglichen. Ein eventuelles Rückspulen ist hier wegen des apparativen Aufwandes nicht vorgesehen und könnte auch zu undefinierten Testmittelzuständen führen. Als nächste Benutzeraktion erfolgt anschließend der Blutauftrag auf das über der Messspitze positionierte Testfeld 44. Sodann kann ein Messergebnis mittels Messeinheit 31 gewonnen und auf der Anzeige 22 angezeigt werden. Der Messzyklus wird auf einfache Weise durch Schließen des Spitzenschutzes 26 durch den Benutzer beendet, wobei das benutzte Testfeld 44 durch weiteren Bandtransport auf der Aufwickelspule 42 entsorgt und das Gerät abgeschaltet wird. Ein solcher Messzyklus lässt sich bis zum Verbrauch aller bevorrateten Testfelder 44 wiederholen, bevor ein Kassettenwechsel notwendig ist.

Sofern im Messbetrieb während eines Messzyklus ein nicht vorgesehener, aber überwachter Gerätezugriff erfolgt, insbesondere das Kassettenfach 34 geöffnet wird, verwirft die Steuereinrichtung aus Sicherheitsgründen den laufenden Test, um eine eventuelle Messwertverfälschung auszuschließen. Hier ist zu bedenken, dass die Gerätebedienung auch für Laien ausgelegt sein muss, und dass ein falsches Messergebnis zu schwerwiegenden Fehldiagnosen führen kann.

Zu unterscheiden davon ist ein notwendiger Zugriff auf das Geräteinnere, um eine Reinigung vorzunehmen oder andere Servicefunktionen bzw. Demonstrationszwecke zu erfüllen. In einem solchen Fall soll ein nachteiliger Verlust eines Testmittels bzw. Testfelds 44 vermieden werden. Hierfür lässt sich die Steuereinrichtung durch einen definierten Steuereingriff in einen Wartungsbetrieb versetzten, wie im rechten Diagrammteil der Fig. 3 gezeigt. Der Steuereingriff wird durch den Menüpunkt "Reinigen" eines Softwaremenüs realisiert, das der Benutzer über die Bedientaste 20 einfach aufrufen kann. Diese Taste ist als "Softkey" ausgelegt und kann somit abhängig von einer Bildschirmanzeige unterschiedliche Funktionen ausüben.

Sobald der Wartungsbetrieb eingeleitet ist, werden die Sensorsignale beim Öffnen des Spitzenschutzes 26 oder des Kassettenfachs 34 ignoriert bzw. nicht mehr als Startaktuation interpretiert, so dass kein automatischer Bandtransport mehr erfolgt. Der Start eines Messzyklus wird somit blockiert, und ein ungewollter Testmittelverlust wird verhindert.

Nun kann der Benutzer über den Bildschirm 22 zur Vornahme bestimmter Handlungen aufgefordert werden. Nach Öffnen des Spitzenschutzes 26 und des Kassettenfachdeckels 36 wird das Gerät abgeschaltet. Der Benutzer kann dann die Kassette 12 entnehmen, den Geräteinnenraum und insbesondere die Messoptik 31 reinigen, die Kassette wieder einlegen und Kassettenfach sowie Spitzenschutz wieder schließen. Das Gerät 10 befindet sich dann wieder in Bereitschaft für den Messbetrieb.

## Patentansprüche

1. Verfahren zur Steuerung eines Analysehandgeräts (10) zur Untersuchung einer Kdrperflüssigkeit, insbesondere für Blutzuckertests, bei dem mittels einer gerätesertigen elektronischen Steuereinrichtung (16) in aufeinanderfolgenden Messzyklen jeweils mindestens ein Testmittel (44) für den Einmalgebrauch automatisch bereitgestellt wird, wobei jeder Messzyklus in einem Messbetrieb der Steuereinrichtung (18) durch eine Startaktuation ausgelöst wird und ein Testband zur Bereitstellung der Testmittel (44) abschnittsweise vorgespult wird, **dadurch gekennzeichnet dass** die Steuereinrichtung (16) durch einen definierten Steuereingriff in einen Wartungsbetrieb versetzt wird, in welchem der automatische Bandtransport unterbrochen wird, so dass die automatische Bereitstellung eines Testmlttels (44) In dem Analysehandgerät (10) verhindert wird.

2. Verfahren nach Anspruch 1, **dadurch gekennzeichnet, dass** der Start eines Messzyklus Im Wartungsbetrieb auch bei einer Startaktuation blockiert wird.

3. Verfahren nach Anspruch 1 oder 2, **dadurch gekennzeichnet, dass** im Wartungabetrieb eine Gerätereinlgung oder ein Testmittelaustausch ohne Testmittelverlust ermöglicht wird.

4. Verfahren nach einem der Ansprüche 1 bis 3, **dadurch gekennzeichnet, dass** der Wartungsbetrieb über ein Softwaremenü (24) der elektronischen Steuereinrichtung (16) durch einen Benutzer ausgewählt wird.

5. Verfahren nach einem der Ansprüche 1 bis 4, **dadurch gekennzeichnet, dass** der Wartungsbetrieb über einen manuell betätigbaren Schalter (20), insbesondere einen Softkey eingeleitet wird.

6. Verfahren nach einem der Ansprüche 1 bis 5, **dadurch gekennzeichnet, dass** eine vorbestimmte Betätigung eines Gerätebauteils durch die Steuereinrichtung (16) als Startaktuation erkannt wird.

7. Verfahren nach einem der Ansprüche 1 bis 6, **dadurch gekennzeichnet, dass** die Startaktuation und/oder ein Austausch der Testmittel (44) durch einen Sensor (37) überwacht wird, und dass im Wartungsbetrieb das Sensorsignal Ignoriert wird.

8. Verfahren nach einem der Ansprüche 1 bis 7, **dadurch gekennzeichnet, dass** die Testmittel (44) in einem Gehäuse (14) des Analysehandgeräts gegen Umweiteinflüsse, insbesondere Feuchtigkeitszutritt geschützt werden.

9. Verfahren nach einem der Ansprüche 1 bis 8, **dadurch gekennzeichnet, dass** die Messzyklen durch das Öffnen einer Schutzabdeckung (26) des Analysehandgeräts ausgelöst werden, wobei ein Testmittel (44) für einen Benutzerzugriff bereitgestellt wird.

10. Verfahren nach einem der Ansprüche 1 bis 9, **dadurch gekennzeichnet, dass** die Testmittel (44) im Messbetrieb durch eine Transporteinrichtung (30) an mindestens eine vorgegebene Position transportiert werden,.

11. Verfahren nach einem der Ansprüche 1 bis 10, **dadurch gekennzeichnet, dass** während eines Messzyklus die jeweiligen Testmittel (44) von einem Testmittelvorrat (40) zu einer Applikationsstelle (46) und gegebenenfalls zu einem Testmittelabfall (42) transportiert werden.

12. Verfahren nach einem der Ansprüche 1 bis 11, **dadurch gekennzeichnet, dass** ein mit einer Vielzahl von analytischen Testfeldern und/oder Stechelementen als Testmittel (44) bestücktes Testband (38) elngesetzt wird, und dass das Testband (38) zur Bereitstellung der Testmittel (44) abschnittsweise vorgespult wird.

13. Verfahren nach einem der Ansprüche 1 bis 12, **dadurch gekennzeichnet, dass** die Testmittel (44) vorzugsweise in Form eines Magazins oder einer Kassette (12) in ein Gerätefach (34) eingewechselt werden, und dass im Meesbetrieb beim Öffnen des Gerätefachs (34) ein durch eine Startaktuation aktiviertes Testmittel (44) verworfen wird.

14. Analysehandgerät zur Untersuchung einer Körperflüssigkeit, insbesondere für Blutzuckertests, mit einer elektronischen Steuereinrichtung (16) zur automatischen Bereitstellung von für den Einmalgebrauch ausgebildeten Testmitteln (44) in aufeinanderfolgenden Mesäzyklen, wobei Jeder Mesaryklus in einem Messbetrieb der Steuereinrichtung (16) durch eine Startaktuation auslösbar ist und ein Testband zur Bereitstellung der Testmittel (44) abschnittsweise vorgespult wird, **dadurch gekennzeichnet, dass** die Steuereinrichtung (16) durch einen definier ten Steuereirigriff in einen Wartungsbetrieb versetzbar ist, in welchem der automatische Bandtransport unterbrochen wird, so dass die automatische Bereitstellung eines Testmittels (44) In dem Analysehandgerät (10) verhindert wird.

## Claims

1. Method for controlling a hand-held analysis device (10) for analysing a body fluid in particular for blood sugar tests in which in each case at least one test means (14) for single use is automatically provided in consecutive measuring cycles by means of an electronic control device (16) implemented in the analysis device, wherein each measuring cycle in a measuring mode of the control device (16) is triggered by a start actuation and a test tape is advanced in sections in order to provide the test means (44), **characterized in that** the control device (16) is put into a maintenance mode by a defined control intervention, in which maintenance mode the automatic tape transport is suspended, so that the automatic provision of a test means (44) in the hand-held analysis device (10) is prevented.

2. Method according to claim 1, **characterized in that** the start of a measuring cycle is blocked in the maintenance mode even in the case of a start actuation.

3. Method according to claim 1 or 2, **characterized in that** the device can be cleaned or test means can be exchanged in the maintenance mode without a loss of test means.

4. Method according to one of the claims 1 to 3, **characterized in that** the maintenance mode is selected by a user via a software menu (24) of the electronic control device (16).

5. Method according to one of the claims 1 to 4, **characterized in that** the maintenance mode is started by a manually operated switch (20) and in particular a softkey.

6. Method according to one of the claims 1 to 5, **characterized in that** a predetermined operation of a device component is recognized by the control device (16) as a start actuation.

7. Method according to one of the claims 1 to 6, **characterized in that** the start actuation and/or an exchange of test means (44) is monitored by a sensor (37) and that the sensor signal is ignored in the maintenance mode.

8. Method according to one of the claims 1 to 7, **characterized in that** the test means (44) are protected in a housing (14) of the hand-held analysis device against environmental influences and in particular the entry of moisture.

9. Method according to one of the claims 1 to 8, **characterized in that** the measuring cycles are triggered by the opening of a protective cover (26) of the hand-held analysis device whereupon a test means (44) is provided for access by the user.

10. Method according to one of the claims 1 to 9, **characterized in that** in the measuring mode the test means (44) are transported by a transport device (30) to at least one predetermined position and that the automatic transport of test means is suspended in the maintenance mode.

11. Method according to one of the claims 1 to 10, **characterized in that** the respective test means (44) are transported from a store of test means (40) to an application site (46) and optionally to a test means waste (42) during a measuring cycle.

12. Method according to one of the claims 1 to 11, **characterized in that** a test tape (38) furnished with a plurality of analytical test fields and/or lancing elements as test means (44) is used and that the test tape (38) is advanced in sections in order to provide the test means (44).

13. Method according to one of the claims 1 to 12, **characterized in that** the test means (44) can be exchanged in a compartment of the device (34) preferably in the form of a magazine or a cassette (12) and that a test means (44) activated by a start actuation is discarded in the measuring mode when the compartment of the device (34) is opened.

14. Hand-held analysis device for analyzing a body fluid in particular for blood sugar tests comprising an electronic control device (16) for automatically providing test means (44) configured for single use in consecutive measuring cycles, wherein each measuring cycle can be triggered in a measuring mode of the control device (16) by a start actuation and wherein a test tape is advanced in sections in order to provide the test means (44), **characterized in that** the control device (16) can be put into a maintenance mode by a defined control intervention, in which maintenance mode the automatic tape transport is suspended, such that the automatic provision of a test means (44) in the hand-held analysis device (10) is prevented.

## Revendications

1. Procédé pour la commande d'un appareil manuel d'analyse (10) pour l'examen d'un liquide corporel, en particulier pour des tests de glycémie, dans lequel à chaque fois au moins un moyen de test (44) est mis à disposition de façon automatique pour un usage unique dans des cycles de mesure consécutifs au moyen d'un dispositif de commande (16) électronique côté appareil, chaque cycle de mesure étant déclenché par une activation de démarrage dans un mode de mesure du dispositif de commande (16) et une bande de test étant avancée par bobinage par sections pour la mise à disposition des moyens de test (44), **caractérisé en ce que** le dispositif de commande (16) est placé par une action de commande défini dans un mode de maintenance, dans lequel le transport de bande automatique est interrompu, de sorte que la mise à disposition automatique d'un moyen de test (44) dans l'appareil manuel d'analyse (10) est empêchée.

2. Procédé selon la revendication 1, **caractérisé en ce que** le démarrage d'un cycle de mesure dans le mode de maintenance est bloqué même dans le cas d'un déclenchement de démarrage.

3. Procédé selon la revendication 1 ou 2, **caractérisé en ce qu'**un nettoyage de l'appareil ou un échange de moyen de test sans perte de moyen de test est rendu possible dans le mode de maintenance.

4. Procédé selon l'une des revendications 1 à 3, **caractérisé en ce que** le mode de maintenance est sélectionné par un utilisateur au moyen d'un menu de logiciel (24) du dispositif de commande (16) électronique.

5. Procédé selon l'une des revendications 1 à 4, **caractérisé en ce que** le mode de maintenance est déclenché au moyen d'un interrupteur (20) pouvant être actionné manuellement, en particulier d'une touche programmable.

6. Procédé selon l'une des revendications 1 à 5, **caractérisé en ce qu'**une commande prédéfinie d'un composant d'appareil est détectée par le dispositif de commande (16) comme déclenchement de démarrage.

7. Procédé selon l'une des revendications 1 à 6, **caractérisé en ce que** le déclenchement de démarrage et/ou un remplacement des moyens de test (44) est/sont contrôlé(s) par un capteur (37) et **en ce que** signal de capteur est ignoré dans le mode de maintenance.

8. Procédé selon l'une des revendications 1 à 7, **caractérisé en ce que** les moyens de test (44) sont protégés dans un boîtier (14) de l'appareil manuel d'analyse contre les influences de l'environnement, en particulier l'arrivée d'humidité.

9. Procédé selon l'une des revendications 1 à 8, **caractérisé en ce que** les cycles de mesure sont déclenchés par l'ouverture d'un revêtement de protection (26) de l'appareil manuel d'analyse, un moyen de test (44) étant mis à disposition pour un accès de l'utilisateur.

10. Procédé selon l'une des revendications 1 à 9, **caractérisé en ce que** les moyens de test (44) sont transportés, dans le mode de mesure, par un dispositif de transport (30) en au moins une position prédéfinie.

11. Procédé selon l'une des revendications 1 à 10, **caractérisé en ce que** les moyens de test (44) respectifs sont transportés pendant un cycle de mesure à partir d'une réserve de moyens de test (40) vers un point d'application (46) et éventuellement jusqu'à un déchet de moyens de test (42).

12. Procédé selon l'une des revendications 1 à 11, **caractérisé en ce qu'**une bande de test (38) équipée d'une pluralité de champs de test analytiques et/ou d'éléments de perforation comme moyens de test (44) est utilisée et **en ce que** la bande de test (38) est avancée par bobinage par sections pour la mise à disposition des moyens de test (44).

13. Procédé selon l'une des revendications 1 à 12, **caractérisé en ce que** les moyens de test (44) sont emballés de préférence sous la forme d'un chargeur ou d'une cassette (12) dans un casier d'appareil (34) et **en ce que**, dans le mode de mesure, un moyen de test (44) activé par une activation de démarrage est rejeté lors de l'ouverture du casier d'appareil (34).

14. Appareil manuel d'analyse pour l'examen d'un liquide corporel, en particulier pour des tests de glycémie, comprenant un dispositif de commande électronique (16) pour la mise à disposition automatique de moyens de test (44) conçus pour un usage unique dans des cycles de mesure consécutifs, chaque cycle de mesure pouvant être déclenché par une activation de démarrage dans un mode de mesure du dispositif de commande (16) et une bande de test étant avancée par bobinage par sections pour la mise à disposition des moyens de test (44), **caractérisé en ce que** le dispositif de commande (16) peut être mis dans un mode de maintenance par une action de commande défini, mode dans lequel le transport de bande automatique est interrompu, de sorte que la mise à disposition automatique d'un moyen de test (44) dans l'appareil manuel d'analyse (10) est empêchée.
